# EUROPÄISCHE PATENTSCHRIFT

(11) **EP 0 496 724 B1**
(45) Veröffentlichungstag und Bekanntmachung des Hinweises auf die Patenterteilung: **15.09.1993**
(21) Anmeldenummer: 92890017.4
(22) Anmeldetag: 22.01.1992
(51) Int. Cl.: A61M 39/00

(54) **Kupplung für die Verbindung von Schlauchleitungen für medizinische Zwecke**
Coupling for joining flexible tubing for medical purposes
Raccord de conduits tubulaires à usage medical

(30) Priorität: 22.01.1991 AT 135/91
(43) Veröffentlichungstag der Anmeldung: 29.07.1992
(73) Patentinhaber: DIERINGER, Franz A., A-1040 Wien (AT)
(72) Erfinder: DIERINGER, Franz A., A-1040 Wien (AT)
(74) Vertreter: Haffner, Thomas M., Dr.

(56) Entgegenhaltungen:
- EP-A- 0 116 986
- EP-A- 0 198 407
- WO-A-90/07953
- AT-A- 386 523

## Beschreibung

Die Erfindung bezieht sich auf eine Kupplung für die Verbindung von Schlauchleitungen für medizinische Zwecke, mit einem ersten Kupplungsteil, in welchem ein axial verschieblicher, Durchtrittsöffnungen aufweisender, hohler Dorn für den Anschluß eines ersten Schlauchstückes gelagert ist, welcher in der Betriebslage einen mit Desinfektionsmittel tränkbaren Schwamm durchsetzt und in einen lösbar mit dem ersten Kupplungsteil verbundenen zweiten Kupplungsteil mit seinen Durchtrittsöffnungen mündet, wobei der zweite Kupplungsteil wenigstens zwei Anschlußöffnungen aufweist.

Eine Einrichtung der eingangs genannten Art ist aus der WO 90/07953 bekanntgeworden. Bei der bekannten sterilen Verbindung von Schlauchleitungen für medizinische Zwecke wird ein hohler Dorn axial verschiebbar in einem Kupplungsteil gehalten und das freie, dem Schlauchanschluß des Dornes abgewandte Ende desselben bewegt sich bei axialer Verschiebung des Dornes durch elastisch verformbare Einlagen, welche mit Desinfektionsmittel imprägniert werden können, in den zweiten Kupplungsteil. Dieser zweite, mit dem ersten Kupplungsteil verbindbare Kupplungsteil weist zwei Öffnungen auf, wovon eine zentrische Entlüftungsöffnung durch einen Fortsatz des Dornes in dessen Endlage der Verschiebung verschlossen wird und über die zweite Öffnung des zweiten Kupplungsteiles Infusionsflaschen, Beutel od.dgl. direkt mit den Patienten verbunden werden. Zum Wechsel derartiger Infusionsflaschen, Beutel od.dgl. war es bei der Einrichtung nach der WO 90/07953 lediglich erforderlich, den Dorn wiederum durch die Lippendichtung und die Abstreifflächen hindurch in seine Ausgangslage zu bewegen, um sicherzustellen, daß während des Wechsels des Infusionsbesteckes od.dgl. ein dichtender Abschluß für die Infusionslösung besteht und somit die Infektionsgefahr vermindert wird. Nach Wechsel der Infusionsflaschen, Beutel od.dgl. konnte der hohle Dorn wiederum in seine Verschiebelage bewegt werden und dem Patienten neuerlich durch Herstellen einer offenen Verbindung zwischen der Infusionsflasche und dem hohlen Dorn Infusionslösungen od.dgl. zugeführt werden. Um bei Verschiebung des Dorns eine Abdichtung der Entlüftungsöffnung zu erzielen, war es bei der bekannten Ausbildung erforderlich, den hohlen Dorn mit seinem Fortsatz unter hoher mechanischer Belastung in die Entlüftungsöffnung zu pressen.

Die Erfindung zielt nun darauf ab, eine Einrichtung der eingangs genannten Art zu schaffen, bei welcher eine Kontamination der Infusionslösungen durch Desinfektionsmittel, Umgebungsluft od.dgl. mit Sicherheit vermieden wird und eine sichere Abdichtung einer Entlüftungsöffnung oder zusätzlichen Anschlußöffnung unter geringer mechanischer Beanspruchung des hohlen Dorns erreicht wird. Zur Lösung dieser Aufgabe besteht die Erfindung im wesentlichen darin, daß der zweite Kupplungsteil ein in Längsrichtung verschiebbares Verschlußstück für eine im wesentlichen mittig angeordnete Anschlußöffnung aufweist, und daß der hohle Dorn eine Anschlagfläche für die Verschiebung des Verschlußstückes aufweist. Dadurch, daß der zweite Kupplungsteil ein in Längsrichtung verschiebbares Verschlußstück für eine im wesentlichen mittig angeordnete Anschlußöffnung aufweist, wird sichergestellt, daß bei axialer Verschiebung des hohlen Dornes die im wesentlichen mittig angeordnete Anschlußöffnung durch das verschiebbare Verschlußstück sicher abgeschlossen wird und der hohle Dorn keinen übermäßigen mechanischen Belastungen, welche bei Verschiebung und Verdrehung des Dornes in der im wesentlichen mittig angeordneten Anschlußöffnung auftreten würden, ausgesetzt wird. Dadurch, daß der hohle Dorn eine Anschlagfläche für die Verschiebung des Verschlußstückes aufweist, kann das Verschlußstück lediglich durch Auflage des hohlen Dorns an seiner der im wesentlichen mittig angeordneten Anschlußöffnung abgewandten Seite axial verschoben werden, wobei bei einer derartigen Verschiebung auf den hohlen Dorn wiederum nur äußerst geringe mechanische Beanspruchungen wirken.

In vorteilhafter Weise wird der zweite Kupplungsteil so ausgeführt, daß das Verschlußstück in Längsrichtung der Kupplung unverdrehbar geführt ist. Dadurch, daß das Verschlußstück in Längsrichtung der Kupplung unverdrehbar geführt ist, wird sichergestellt, daß sich das Verschlußstück bei Verschiebung in Längsrichtung nicht in dem zweiten Kupplungsteil in unerwünschter Weise verkeilen oder kippen kann und so ein Abschluß der im wesentlichen mittig angeordneten Anschlußöffnung nicht oder nicht vollständig sichergestellt wird. Durch die unverdrehbare Führung des Verschlußstückes in Längsrichtung der Kupplung wird ein sicherer Abschluß der mittig angeordneten Anschlußöffnung ohne wesentlichen Kraftaufwand und ohne größere mechanische Belastungen, sowohl des Verschlußstückes als auch des hohlen Dornes sichergestellt. Die unverdrehbare Führung des Verschlußstückes in Längsrichtung der Kupplung wird in besonders vorteilhafter Weise dadurch sichergestellt, daß das Verschlußstück wenigstens einen im wesentlichen radialen Fortsatz aufweist, welcher in eine in Längsrichtung verlaufende Ausnehmung oder Nut am Innenmantel des zweiten Kupplungsteiles eingreift. Durch Eingriff des im wesentlichen radialen Fortsatzes in eine in Längsrichtung verlaufende Ausnehmung oder Nut am Innenmantel des zweiten Kupplungsteiles wird eine Führung für die Verschiebung des Verschlußstückes in axialer Richtung erzielt und dadurch auch ein sicherer Verschluß der im wesentlichen mittig angeordneten Anschlußöffnung des zweiten Kupplungsteiles sichergestellt. Um neben einem sicheren Abschluß der im wesentlichen mittig angeordneten Anschlußöffnung auch einen dichten Abschluß dieser Anschlußöffnung sicherzustellen, ist die Erfindung in vorteilhafter Weise so weitergebildet, daß das Verschlußstück einen, insbesonderen konischen, Fortsatz aufweist, welcher mit der mittig angeordneten, insbesondere hohlkonisch, ausgebildeten Anschlußöffnung zusammenwirkt, wobei in besonders bevorzugter Ausführung die mit dem Fortsatz zusammenwirkende Anschlußöffnung an ihrem Innenumfang ein Dichtelement trägt. Dadurch, daß das Verschlußstück einen konischen Fortsatz aufweist, welcher mit der entsprechend hohlkonisch ausgebildeten Anschlußöffnung, welche an ihrem Innenumfang ein Dichtelement trägt, zusammenwirkt, wird das Zentrieren zur Erzielung eines dichten Abschlusses der im wesentlichen mittig angeordneten Anschlußöffnung bei gleichzeitiger geringer mechanischer Belastung oder Dichtung sichergestellt, wobei durch einen derartigen dichten Abschluß der im wesentlichen mittig angeordneten Anschlußöffnung der Eintritt von die Infusionslösungen kontaminierenden Stoffen, wie beispielsweise Fremdpartikel oder Umgebungsluft, mit Sicherheit vermieden wird.

In besonders vorteilhafter Weise wird der zweite Kupplungsteil so ausgebildet, daß die mit dem Fortsatz zusammenwirkende Anschlußöffnung wenigstens ein Verriegelungsglied, insbesondere sägezahnförmige Vorsprünge, aufweist, welches mit entsprechenden Verriegelungsgliedern des Verschlußstückes zusammenwirkt. Dadurch, daß die mit dem Fortsatz zusammenwirkende Anschlußöffnung ein Verriegelungsglied aufweist, welches mit entsprechenden Verriegelungsgliedern des Verschlußstückes zusammenwirkt, wird eine dichte und unlösbare Verbindung des Verschlußstückes mit der im wesentlichen mittig angeordneten Anschlußöffnung erreicht. Eine derartige, unlösbare Verbindung des Verschlußstückes mit der im wesentlichen mittig angeordneten Anschlußöffnung stellt sicher, daß das Verschlußstück nach einem einmal erzielten Abschluß der mittigen Anschlußöffnung nicht wieder geöffnet werden kann und somit zumindest der zweite Kupplungsteil lediglich ein einziges Mal verwendet werden kann. Eine derartige Verhinderung einer Wiederverwendung des zweiten Kupplungsteiles verringert das Risiko, daß in die Kupplung kontaminierende Stoffe durch die im wesentlichen mittig angeordnete Anschlußöffnung bei einem Zurückziehen des Dornes und einem Wechsel von Behältern für Infusionsflüssigkeiten eingetragen werden, und stellt so sicher, daß während der Zufuhr von Infusionslösungen absolut sterile Bedingungen in der Kupplung aufrechterhalten werden.

Um eine einfache und sichere Verschiebung des Verschlußstückes mit Hilfe des hohlen Dornes in dem Innenteil des zweiten Kupplungsteiles sicherzustellen, ist die erfindungsgemäße Einrichtung im wesentlichen so ausgebildet, daß das Verschlußstück an seiner der Anschlußöffnung abgewandten Stirnfläche eine Ausnehmung oder einen Fortsatz aufweist, welche(r) mit einem Fortsatz oder einer Ausnehmung des hohlen Dornes zusammenwirkt. Dadurch, daß das Verschlußstück an seiner der Anschlußöffnung abgewandten Stirnfläche eine Ausnehmung oder einen Fortsatz aufweist, welche(r) mit einem Fortsatz oder einer Ausnehmung des hohlen Dornes zusammenwirkt, wird sichergestellt, daß auch ein Verkippen oder Verkeilen des hohlen Dornes bei einer axialen Verschiebung entlang der Kupplung mit Sicherheit vermieden wird und eine exakte gegenseitige Lage der verschiebbaren Elemente eingehalten wird.

In vorteilhafter Weise ist der zweite Kupplungsteil so ausgebildet, daß das Verschlußstück in der OFFEN-Stellung in Abstand von der Anschlußöffnung dichtend gelagert ist, wobei die Lagerung in vorteilhafter Weise eine, eine ringförmige Dichtung aufweisende Durchtrittsöffnung aufweist. Durch eine dichtende Lagerung des Verschlußstückes in der OFFEN-Stellung wird der im oberen Teil des zweiten Kupplungsstückes gebildete Raum gegen die von den Anschlußöffnungen abgewandte Seite abgedichtet, und es kann der sich im oberen Teil des zweiten Kupplungsstückes befindende Raum mit Infusionslösung oder anderen, dem Patienten zuführenden Pharmazeutika ohne Einschluß von Luftblasen befüllt werden, bevor das Verschlußstück in seine GESCHLOSSEN-Stellung verschoben wird. Bei einer Anpassung der jeweiligen Außendurchmesser des hohlen Dornes und des Verschlußstückes wird auch für eine abgedichtete Lage des Dornes in der Betriebsstellung Sorge getragen.

In vorteilhafter Weise ist die erfindungsgemäße Kupplung so ausgebildet, daß der hohle Dorn in seiner der Schließstellung des Verschlußstückes entsprechenden Verschiebelage über ein radiales, die Wand des ersten Kupplungsteiles durchsetzendes Betätigungsglied verdrehbar in einer Kulisse geführt ist, und daß die Kulisse in einer zu einer Normalebene auf die Achse des Dornes geneigten Ebene liegt. Dadurch, daß der hohle Dorn mit Hilfe eines die Wand des ersten Kupplungsteiles durchsetzenden Betätigungsgliedes verdrehbar in einer Kulisse geführt wird, wird nach axialer Verschiebung und Verdrehung des Dornes in der Kulisse ein Zurückgleiten des Dornes in seine Ausgangslage mit Sicherheit vermieden. Dadurch, daß die Kulisse in einer zu einer Normalebene auf die Achse des Dornes geneigten Ebene liegt, wird ein fester Eingriff des Fortsatzes oder des Ausnehmung des hohlen Dornes in dem Verschlußglied und damit ein sicherer Eingriff des Fortsatzes des Verschlußstückes in der im wesentlichen mittig angeordneten Anschlußöffnung des zweiten Kupplungsteiles sichergestellt und somit eine Fixierung der OFFEN-Stellung der gesamten Kupplung erreicht.

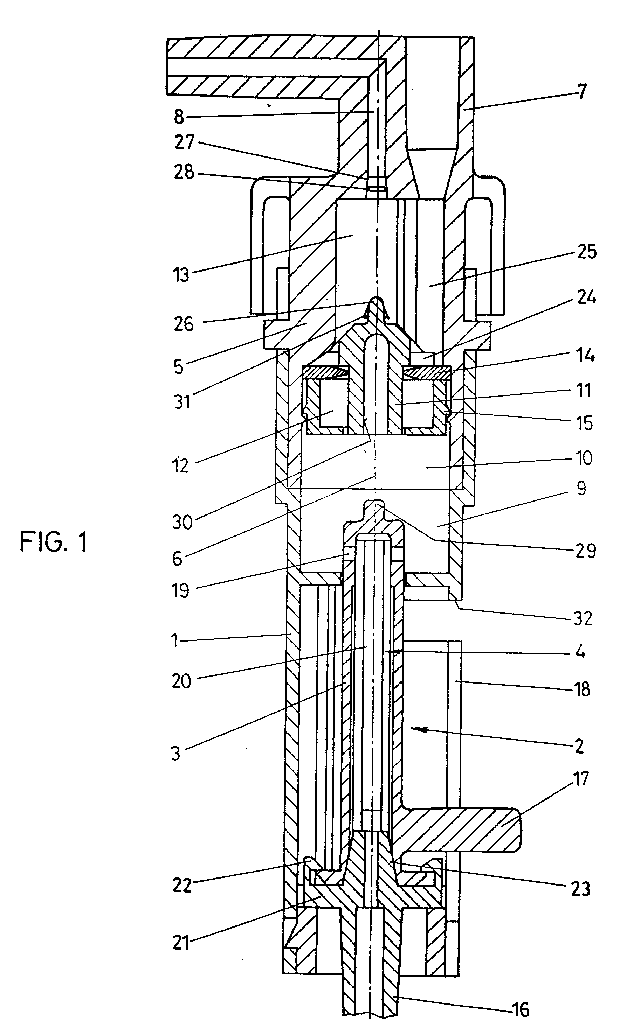

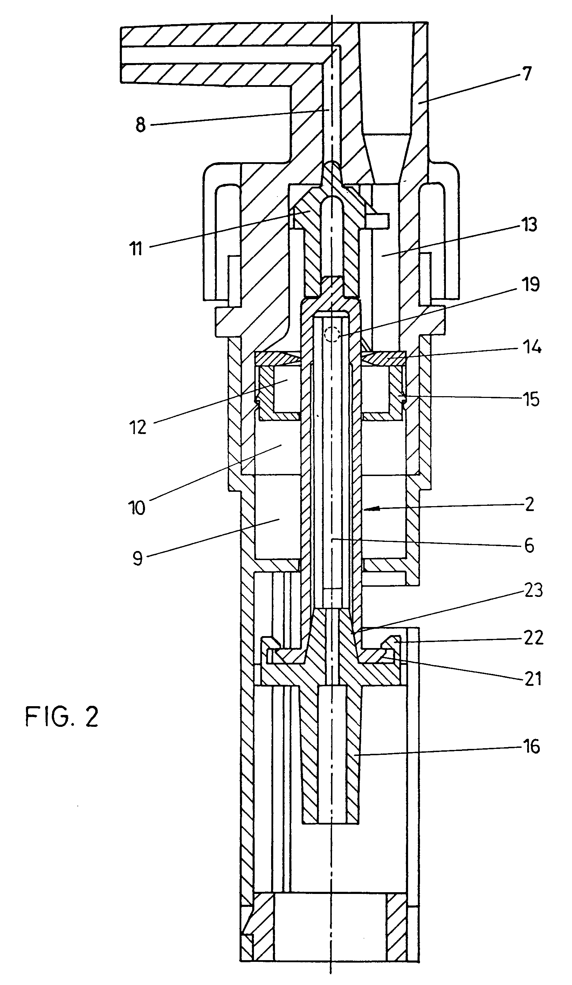

Die Erfindung wird nachfolgend an Hand eines in der Zeichnung dargestellten Ausführungsbeispieles näher erläutert. In dieser zeigen: Fig.1 eine schematische Darstellung der erfindungsgemäßen Kupplung im Axialschnitt in der AUSSERBETRIEB-Stellung, und Fig.2 eine zu Fig.1 analoge Darstellung mit dem hohlen Dorn in BETRIEB-Stellung.

In Fig.1 ist ein erster Kupplungsteil 1 mit einem in Achsrichtung verschieblichen Dorn 2 dargestellt, wobei dieser Dorn 2 aus einem Außenteil 3 und einem Innenteil 4 besteht. An diesen ersten Kupplungsteil 1 ist ein zweiter Kupplungsteil 5 angeschlossen, an welchem exzentrisch zur Achse 6 des Dornes bzw. des Verschiebeweges des Dornes ein Anschluß 7 für eine weiterführende Schlauchleitung zu einem Infusionsbesteck vorgesehen ist. Zentrisch und innerhalb der Achse 6 ist am zweiten Kupplungsteil 5 eine Entlüftungsöffnung 8 vorgesehen. Bei einer axialen Verschiebung des Dornes 2 wird der Dorn 2 zunächst durch eine erste Einlage 9 aus saugfähigem Material hindurchbewegt, welche permanent mit Desinfektionsmittel getränkt ist. In axialer Richtung anschließend ist eine beispielsweise austauschbare, zweite, saugfähige und im zweiten Kupplungsteil aufgenommene Einlage 10 vorgesehen, in welcher Desinfektionsmittel ergänzt werden kann, um eine sichere Desinfektion zu gewährleisten. Im zweiten Kupplungsteil 5 ist ein in Längsrichtung derselben verschiebbares Verschlußstück 11 für die Öffnung 8 sowie eine weitere saugfähige Einlage 12 vorgesehen, welche an der Außenseite des Dornes 2 anhaftendes Material abstreift und aufsaugt.

Beim Anschluß einer Infusionsflasche an den Anschluß 7 kann eine Entlüftung dadurch erzielt werden, daß das eintretende Medium solange in den Raum 13 eingebracht wird, bis es über die Entlüftungsbohrung 8 wiederum abströmt. Um bei einem derartigen Spülvorgang zur Entfernung von Restluft sicherzustellen, daß die Abstreifeinlage 12 nicht mit dem Medium in Berührung gelangt und somit nicht mit Medium getränkt wird, wodurch sie ihre Saugfähigkeit behält, ist das Verschlußstück 11 in einer ringförmigen Dichtung 14 gehalten. Die Abstreifscheibe 12 sowie die Dichtungsscheibe 14 sind in einem gemeinsamen Bauteil 15 gehalten, welcher mit dem zweiten Kupplungsteil 5 verrastbar ist.

Um in zurückgezogener Stellung des Dornes, wie dies in Fig.1 dargestellt ist, sicherzustellen, daß ein dichtender Abschluß der an den Dorn über den Anschluß 16 angeschlossenen, zum Patienten führenden Leitung gewährleistet ist, ist der äußere Teil 3 des Dornes 2 mit einem radialen Betätigungsfortsatz 17 ausgestattet, welcher eine Führung in der Wand des ersten Kupplungsteiles 1 durchsetzt. Der innere Dornteil 4 steht mit dem Anschluß 16 in Verbindung, wobei in der in Fig.1 dargestellten Drehlage der innere Dornteil 4 vom Außenteil 3 des Dornes 2 dichtend umgriffen wird, so daß in der in Fig.1 dargestellten Lage die radialen Durchbrechungen 19 des Außenteiles 3 des Dornes abgedichtet sind.

Der Innendorn 4 weist in axialer Richtung verlaufende Kanäle 20 auf, welche in jeweils vorgegebener Drehlage mit den radialen Durchbrechungen 19 des Außenteiles 3 des Dornes in fluchtende Stellung gebracht werden können. Der Innenteil 4 des Dornes ist mit einem Basisteil 21 verbunden, welcher mit Klauen 22 für die Verriegelung des Außenteiles 3 des Dornes 2 ausgestattet ist. Über den Basisteil 21 mündet der Anschluß 16 der zum Patienten führenden Leitung in den in axialer Richtung verlaufenden Raum, welcher von den Ausnehmungen 20 des Innenteiles des Dornes 4 und der Innenwand des Außenteiles 3 des Dornes begrenzt ist. Der Basisteil weist weiters eine konische Dichtfläche 23 auf, welche nach dem Aufstecken des Außenteiles des Dornes in eine durch die Klauen 22 des Basisteiles 21 gegen axiale Verschiebung gesicherte Raststellung eine Dichtfläche zum Außenteil 3 des Dornes ausbilden.

Das gesonderte und in axialer Richtung der Kupplung verschiebbare Verschlußstück 11 wird der in Fig.1 gezeigten AUSSERBETRIEB-Stellung im hülsenförmigen Bauteil durch die Dichtung 14 gehalten und ist im zweiten Kupplungsteil 5 unverdrehbar geführt. Zu diesem Zweck weist das Verschlußstück 11 einen im wesentlichen radialen Fortsatz 24 auf, welcher mit einer im wesentlichen in axialer Richtung verlaufenden Ausnehmung bzw. Nut 25 an der Innenfläche des zweiten Kupplungsstückes zusammenwirkt, wobei die Nut beim gezeigten Ausführungsbeispiel mit dem Anschluß 7 zu einer, nicht näher dargestellten, Infusionsflasche fluchtet. Das Verschlußstück 11 weist weiters einen konischen Fortsatz 26 auf, welcher bei einer Verschiebung des Verschlußstückes in die Entlüftungsöffnung bzw. im wesentlichen mittig angeordnete Öffnung 8 eintaucht, wobei die Öffnung 8 einen an die Konizität des Fortsatzes 26 angepaßten konischen Bereich 27 aufweist, welcher zur Verbesserung der Dichtwirkung weiters eine Dichtung 28 trägt. Um ein ordnungsgemäßes Zusammenwirken des hohlen Dornes bei der Bewegung des Dornes 2 mit dem Verschlußstück 11 zu bewirken, weist der hohle Dorn an seinem, dem Verschlußstück zugewandten Ende einen Fortsatz 29 auf, welcher in eine entsprechend ausgebildete Ausnehmung bzw. Öffnung 30 des Verschlußstückes 11 eintaucht. Dadurch wird die geradlinige Führung des Verschlußstückes 11 im zweiten Kupplungsteil bei der Verschiebebewegung des Dornes 2 unterstützt. Um eine mehrfache Verwendung des zweiten Kupplungsteiles nach einem Ersatz von Infusionsbehältern zu vermeiden, kann beispielsweise vorgesehen sein, daß der Fortsatz 26 des Verschlußstückes 11 Verriegelungsglieder 31 trägt, welche bei einem erstmaligen Einschieben des axialen Dornes, wie dies in Fig. 2 dargestellt ist, in, nicht näher dargestellten, entsprechenden Ausnehmungen der Anschlußöffnung 8 einrasten, so daß auch bei einem Zurückziehen des Dornes 2 das Verschlußstück 11 in der in Fig . 2 dargestellten Lage verbleibt und für eine Weiterverwendung zumindest das zweite Kupplungsstück getauscht werden muß.

Um in der eingeschobenen Stellung des Dornes 2 zu verhindern, daß in dem Raum 13 befindliche Infusionslösung bzw. Medikamente in den Bereich der mit Desinfektionsmittel getränkten bzw. zum Abstreifen desselben dienenden Schwämme 9, 10 bzw. 12 eindringen, kann für eine geeignete Aufrechterhaltung der Dichtwirkung der Dichtung 14 der Außendurchmesser des äußeren Teiles 3 des Dornes geringfügig größer sein als der Außendurchmesser des im Fortsatz 26 abgewandten Bereiches des Verschlußstückes 11.

Wie oben bereits angedeutet, wird der axiale Dorn 2 über einen Hebel 17 betätigt, welcher in einem ersten Teilbereich 18 einer Kulisse in im wesentlicher axialer Richtung der Kupplung bewegt wird, wobei sich an diesen ersten Teilbereich 18 ein im wesentlichen normal dazu verlaufender Teilbereich 32 des Kulissenführung anschließt, wobei durch die Verdrehung des äußeren Teiles 3 des hohlen Dornes 2 über Betätigung des Hebels 17 die im wesentlichen radialen Bohrungen 19 in die strichliert in Fig. 2 angedeutete Stellung gelangen, wodurch eine Verbindung zwischen dem Raum 13 und den im wesentlichen in Achsrichtung verlaufenden Kanälen bzw. Ausnehmungen 20 des Innenteiles 4 des Dornes hergestellt wird. Um eine gezielte und definierte Einpassung des Verschlußstückes 11 in die Belüftungsöffnung 8 erzielen zu können, verläuft dabei der Teilbereich 32 der Kulissenführung in einer zu einer Normalebene auf die Achse 6 des Dornes 2 geneigten Ebene, wobei die Neigung in Richtung zum zweiten Kupplungsstück verläuft, so daß bei der Verdrehung des Dornes eine weitere geringfügige, axiale Bewegung in Richtung zum zweiten Kupplungsteil erfolgt und somit das Verschlußstück in die Aschlußöffnung eingepreßt wird.

Über die im wesentlichen zentrisch angeordnete Öffnung 8 kann nicht nur eine Belüftung vorgenommen werden, sondern es können auch zusätzliche Medikamente in den Raum 13 eingebracht werden, welche in weiterer Folge dem Patienten zugeführt werden sollen.

## Patentansprüche

1. Kupplung für die Verbindung von Schlauchleitungen für medizinische Zwecke, mit einem ersten Kupplungsteil (1), in welchem ein axial verschieblicher, Durchtrittsöffnungen (19) aufweisender, hohler Dorn (2) für den Anschluß eines ersten Schlauchstückes gelagert ist, welcher in der Betriebslage einen mit Desinfektionsmittel tränkbaren Schwamm (9) durchsetzt und in einen lösbar mit dem ersten Kupplungsteil (1) verbundenen zweiten Kupplungsteil (5) mit seinen Durchtrittsöffnungen (19) mündet, wobei der zweite Kupplungsteil (5) wenigstens zwei Anschlußöffnungen (7,8) aufweist, dadurch gekennzeichnet, daß der zweite Kupplungsteil (5) ein in Längsrichtung verschiebbares Verschlußstück (11) für eine im wesentlichen mittig angeordnete Anschlußöffnung (8) aufweist, und daß der hohle Dorn (2) eine Anschlagfläche (29) für die Verschiebung des Verschlußstückes (11) aufweist.

2. Kupplung nach Anspruch 1, dadurch gekennzeichnet, daß das Verschlußstück (11) in Längsrichtung der Kupplung unverdrehbar geführt ist.

3. Kupplung nach Anspruch 1 oder 2, dadurch gekennzeichnet, daß das Verschlußstück (11) wenigstens einen im wesentlichen radialen Fortsatz (24) aufweist, welcher in eine in Längsrichtung verlaufende Ausnehmung oder Nut (25) am Innenmantel des zweiten Kupplungsteiles (5) eingreift.

4. Kupplung nach Anspruch 1, 2 oder 3, dadurch gekennzeichnet, daß das Verschlußstück (11) einen, insbesonderen konischen, Fortsatz (26) aufweist, welcher mit der mittig angeordneten, insbesondere hohlkonisch, ausgebildeten Anschlußöffnung (8) zusammenwirkt.

5. Kupplung nach einem der Ansprüche 1 bis 4, dadurch gekennzeichnet, daß die mit dem Fortsatz (26) zusammenwirkende Anschlußöffnung (8) an ihrem Innenumfang ein Dichtelement (28) trägt.

6. Kupplung nach einem der Ansprüche 1 bis 5, dadurch gekennzeichnet, daß die mit dem Fortsatz (26) zusammenwirkende Anschlußöffnung (8) wenigstens ein Verriegelungsglied, insbesondere sägezahnförmige Vorsprünge, aufweist, welches mit entsprechenden Verriegelungsgliedern (31) des Verschlußstückes zusammenwirkt.

7. Kupplung nach einem der Ansprüche 1 bis 6, dadurch gekennzeichnet, daß das Verschlußstück (11) an seiner der Anschlußöffnung (8) abgewandten Stirnfläche eine Ausnehmung (30) oder einen Fortsatz aufweist, welche(r) mit einem Fortsatz (29) oder einer Ausnehmung des hohlen Dornes (2) zusammenwirkt.

8. Kupplung nach einem der Ansprüche 1 bis 7, dadurch gekennzeichnet, daß das Verschlußstück (11) in der OFFEN-Stellung in Abstand von der Anschlußöffnung (8) dichtend gelagert ist.

9. Kupplung nach Anspruch 8, dadurch gekennzeichnet, daß die Lagerung (8) eine eine ringförmige Dichtung (28) aufweisende Durchtrittsöffnung aufweist.

10. Kupplung nach einem der Ansprüche 1 bis 9, dadurch gekennzeichnet, daß der hohle Dorn (2) in seiner der Schließstellung des Verschlußstückes (11) entsprechenden Verschiebelage über ein radiales, die Wand des ersten Kupplungsteiles (1) durchsetzendes Betätigungsglied (17) verdrehbar in einer Kulisse geführt ist, und daß die Kulisse in einer zu einer Normalebene auf die Achse des Dornes (2) geneigten Ebene liegt.

## Claims

1. Coupling for the connection of flexible tube lines for medical purposes, having a first coupling part (1) in which there is mounted an axially displaceable hollow pin (2) which has through-openings (19) and is intended for connection of a first flexible tube piece, which pin, in the operating position, passes through a sponge (9), which can be soaked with disinfectant, and opens, with its through-openings (19), into a second coupling part (5) which is releasably connected to the first coupling part (1) and has at least two connection openings (7, 8), characterised in that the second coupling part (5) has a closure piece (11) which can be displaced in the longitudinal direction and is intended for an essentially centrally arranged connection opening (8), and in that the hollow pin (2) has a stop surface (29) for the displacement of the closure piece (11).

2. Coupling according to Claim 1, characterised in that the closure piece (11) is guided in the longitudinal direction of the coupling such that it cannot rotate.

3. Coupling according to Claim 1 or 2, characterised in that the closure piece (11) has at least one essentially radial projection (24) which engages in a longitudinally running cutout or groove (25) on the inner casing of the second coupling part (5).

4. Coupling according to Claim 1, 2 or 3, characterised in that the closure piece (11) has a projection (26) which is, in particular, conical and interacts with the centrally arranged connection opening (8) which is, in particular, in the form of a hollow cone.

5. Coupling according to one of Claims 1 to 4, characterised in that the connection opening (8) interacting with the projection (26) bears a sealing element (28) on its inner circumference.

6. Coupling according to one of Claims 1 to 5, characterised in that the connection opening (8) interacting with the projection (26) has at least one locking member, in particular protrusions in the form of saw teeth, which interacts with corresponding locking members (31) of the closure piece.

7. Coupling according to one of Claims 1 to 6, characterised in that, on its end face directed away from the connection opening (8), the closure piece (11) has a cutout (30) or a projection, which cutout or projection interacts with a projection (29) or a cutout of the hollow pin (2).

8. Coupling according to one of Claims 1 to 7, characterised in that, in the OPEN position, the closure piece (11) is mounted in a sealing manner at a distance from the connection opening (8).

9. Coupling according to Claim 8, characterised in that the mounting (8) has a through-opening which has an annular seal (28).

10. Coupling according to one of Claims 1 to 9, characterised in that, in its displacement position corresponding to the CLOSED position of the closure piece (11), the hollow pin (2) is rotatably guided in a slotted link via a radial actuating member (17) which passes through the wall of the first coupling part (1), and in that the slotted link is located in a plane which is inclined with respect to a plane normal to the axis of the pin (2).

## Revendications

1. Raccord de conduits tabulaires à usage médical, comportant une première partie (1) de raccord, dans laquelle une broche creuse (2), qui est déplaçable axialement et comporte des ouvertures de passage (19), est montée pour le raccordement d'un premier élément de tuyau, la broche traversant, dans la position de fonctionnement, une éponge (9) pouvant être imprégnée d'un agent désinfectant et débouchant par ses ouvertures de passage (19), dans une seconde partie (5) du raccord, qui est réunie de façon amovible à la première partie (1) du raccord, la seconde partie (5) du raccord comportant au moins deux ouvertures de raccordement (7,8), caractérisé en ce que la seconde partie (5) du raccord possède un élément d'obturation (11), qui peut être déplacé dans la direction longitudinale, pour une ouverture de raccordement (8) disposée sensiblement en position centrée, et en ce que la tige creuse (2) possède une surface de butée (29) pour le déplacement de l'élément d'obturation (11).

2. Raccord selon la revendication 1, caractérisé en ce que l'élément d'obturation (11) est guidé, avec blocage en rotation, dans la direction longitudinale du raccord.

3. Raccord suivant la revendication 1 ou 2, caractérisé en ce que l'élément d'obturation (11) possède au moins un prolongement sensiblement radial (24), qui s'engage dans un évidement ou une rainure (25), qui s'étend dans la direction longitudinale et est ménagé dans l'enveloppe intérieure de la seconde partie (5) du raccord.

4. Raccord selon la revendication 1, 2 ou 3, caractérisé en ce que l'élément d'obturation (11) possède un prolongement (26), notamment conique, qui coopère avec l'ouverture de raccordement centrée (8), qui possède notamment une forme conique creuse.

5. Raccord selon l'une des revendications 1 à 4, caractérisé en ce que l'ouverture de raccordement (8), qui coopère avec le prolongement (26), porte un élément d'étanchéité (28) sur son pourtour intérieur.

6. Raccord selon l'une des revendications 1 à 5, caractérisé en ce que l'ouverture de raccordement (8), qui coopère avec le prolongement (26), possède au moins un élément de verrouillage, notamment des éléments saillants en forme de dents de scie, qui coopère avec des éléments de verrouillage correspondants (31) de l'élément de fermeture.

7. Raccord selon l'une des revendications 1 ) 6, caractérisé en ce que l'élément d'obturation (11) comporte, sur sa face frontale tournée à l'opposé de l'ouverture de raccordement (8), un évidement (30) ou un prolongement, qui coopère avec un prolongement (29) ou un évidement de la tige creuse (2).

8. Raccord selon l'une des revendications 1 à 7, caractérisé en ce que dans la position OUVERTE, l'élément d'obturation (11) est supporté, de manière à établir l'étanchéité, à distance de l'ouverture de raccordement (8).

9. Raccord selon la revendication 8, caractérisé en en ce que le support (8) possède une ouverture de passage comportant une garniture d'étanchéité annulaire (28).

10. Raccord selon l'une des revendications 1 à 9, caractérisé en ce que dans sa position décalée correspondant à la position d'obturation de l'élément d'obturation (11), le manchon creux (2) est guidé sous torsion dans une coulisse, par l'intermédiaire d'un élément radial d'actionnement (17) qui traverse la paroi de la première partie (1) du raccord, et en ce que la coulisse est située dans un plan incliné par rapport à un plan normal à l'axe de la broche (2).
